# EUROPEAN PATENT SPECIFICATION

(11) **EP 2 544 628 B1**
(45) Date of publication and mention of the grant of the patent: **22.07.2015**
(21) Application number: 11713886.7
(22) Date of filing: 09.03.2011
(51) Int. Cl.: A61F 2/30

(54) **PROSTHESIS WITH DIFFERENTIATED BIOLOGICAL RESPONSE**
PROTHESE MIT DIFFERENZIERTER BIOLOGISCHER REAKTION
PROTHÈSE AVEC RÉPONSE BIOLOGIQUE DIFFÉRENTIÉE

(30) Priority: 09.03.2010 IT PD20100070
(43) Date of publication of application: 16.01.2013
(73) Proprietor: Sintea Plustek S.r.l., 20090 Assago, Milano (IT)
(72) Inventor: GIULIANI, Alessio, I-20090 Assago, Milano (IT); GUERRA, Paolo, I-20090 Assago, Milano (IT)
(74) Representative: Postiglione, Ferruccio
(86) International application number: PCT/IT2011/000062
(87) International publication number: WO 2011/111083

(56) References cited:
- EP-A1- 1 025 815
- EP-A1- 2 036 517
- WO-A1-03/068286
- WO-A1-2008/115883
- WO-A2-2006/096793
- DE-A1-102006 044 415
- US-A1- 2007 142 916

## Description

The present invention relates to a prosthesis and in particular an orthopaedic prosthesis with differentiated biological response.

In particular, it is known of in the prosthetics sector, to implant orthopaedic prostheses trying to improve osteo-integration, that is to say the bony encapsulation of the prosthesis, as far as possible. Osteo-integration is essential for restoring the biomechanical functions of the prosthesized bone as soon as possible.

Not infrequently however a need arises to service the prosthesis implanted: in practice this entails explanting the prosthesis to replace, reposition or modify it so as to adapt it to the specific anatomical conformation of the patient.

The phase of explanting the prosthesis is extremely invasive in that the prosthesis, thanks to its osteo-integration, is strongly bound both to the cortex and to the inner spongy part of the bone.

Consequently, the removal of prostheses performed according to the prior art is often excessively invasive for the patient.

It follows that in the art, servicing operations are reduced to a minimum except where strictly necessary.

There is therefore a tendency to leave the first prostheses implanted in the patient in position which does not always ensure optimal functioning for the patient. The closest prior art is described in WO 03/068286 and EP 1025815.

The purpose of the present invention is to make a prosthesis which overcomes the drawbacks mentioned with reference to the prior art.

Such drawbacks and limitations are resolved by a prosthesis according to claim 1.

Other embodiments of the prosthesis according to the invention are described in the subsequent claims.

Further characteristics and advantages of the present invention will be clearly comprehensible from the description given below of its preferred embodiments, made by way of a non-limiting example, wherein:
figure 1 shows a perspective view of a prosthesis according to one possible embodiment of the present invention;
figure 2 shows a perspective view of the prosthesis in figure 1 in an implant configuration on an associable bone;
figure 3 shows a perspective view of a prosthesis according to a further embodiment of the present invention, in an implant configuration;
figures 4-10 show enlarged views of parts of the prostheses in figures 1 and 3 according to different embodiments of the present invention.

The elements or parts of elements common to the embodiments described below will be indicated using the same reference numerals.

With reference to the aforesaid figures, reference numeral 4 globally denotes an orthopaedic prosthesis.

The definition of orthopaedic prosthesis should be considered in the broad, not limited sense; in other words any prosthesis suitable to interface with or to replace at least partially a bone or portion of bone.

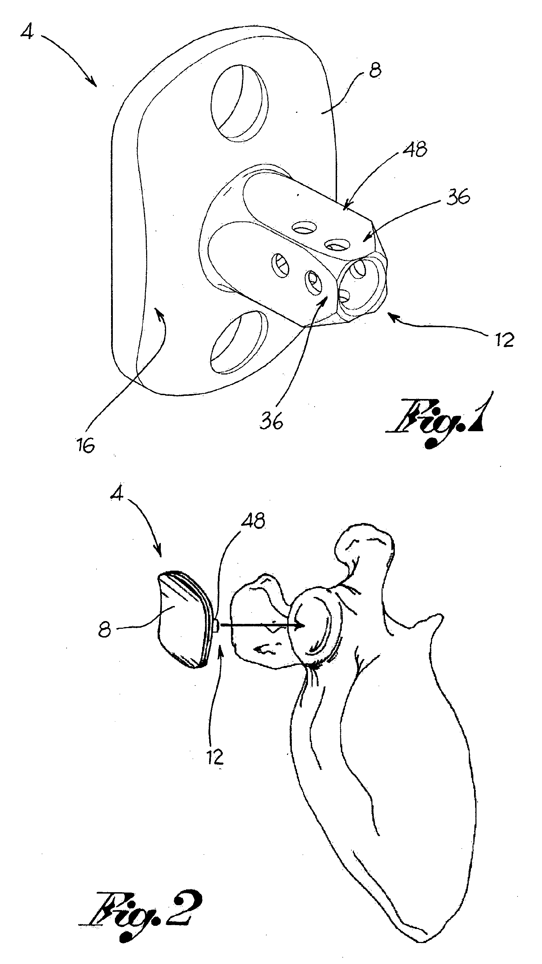

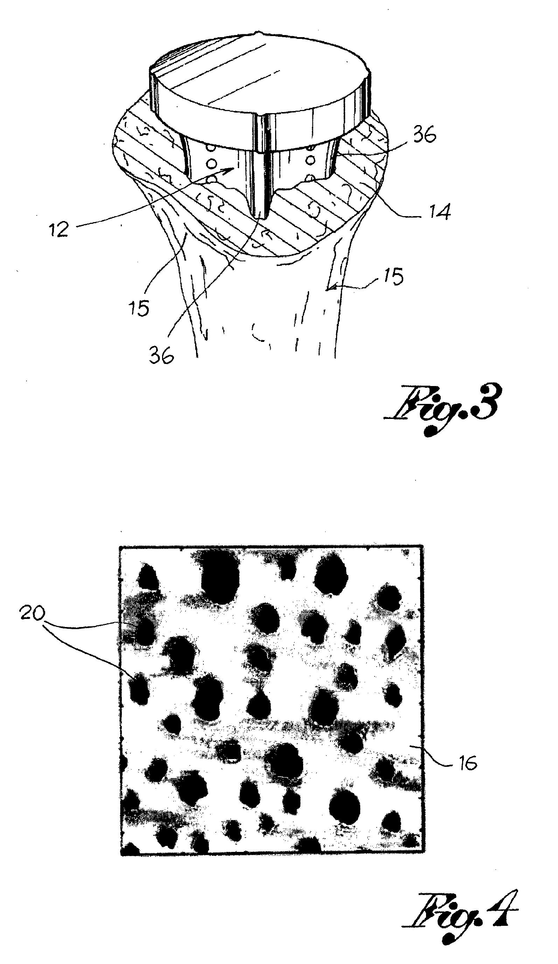

Merely by way of example, one might mean a glenoid (figures 1-2), shoulder (figure 3), knee, hip, or cotyle prosthesis, a prosthesis of a vertebral body and the like.

The orthopaedic prosthesis 4 comprises a prosthesis body 8 suitable to interface with at least one bone or bony tissue; the term "interface" is taken to mean that the prosthesis body 8, or a portion of it, is suitable to position itself in contact with a bone and in particular with a cortex of the bone, that is the external, rigid portion covering the bone.

The prosthesis body 8 comprises at least one primary attachment element 12 for the mechanical attachment of the prosthesis 4 inside the spongy tissue 14 of the bone.

In particular, the spongy tissue 14 is enclosed inside a bone cortex 15 and is thus defined because its consistency is decidedly inferior to that of the bone cortex 15.

Primary attachment is understood to mean the temporary mechanical attachment achieved at the moment of implanting the prosthesis, before any osteo-integration has taken place. The primary attachment of the prosthesis usually occurs by using attachment elements which penetrate inside the bone to at least temporarily attach the prosthesis until the bone grows around it.

Advantageously, the prosthesis body 8 comprises at least one bone grafting wall 16 suitable, in an implant configuration, to be placed in contact with the bone cortex, penetrating at least partially inside the bone cortex 15, said bone grafting wall 16 being hydrophilic so as to improve osteo-integration of the prosthesis 4 with the associable bone.

Thanks to the fact of being hydrophilic, the bone grafting wall 16 ensures the absorption of proteins and thereby the adhesion of osteoblasts; thereby improving and speeding up bone grafting.

According to one embodiment, the bone grafting wall 16 comprises a surface grid having a plurality of recesses 20 of a depth of 14 - 29 nm (figure 4). Preferably said surface grid is irregular; in.other words the recesses 20 are staggered so as not to be aligned along rows and/or columns.

Preferably, said recesses 20 are blind holes not communicating with each other.

According to a further embodiment, the bone grafting wall 16 comprises fibres 24 having a radius of 90 - 110 nm; preferably the fibres 24 have a radius of 100 nm.

Such dimensions in fact enable the cellular adhesion force of the fibres to achieve the theoretical value of Van der Walls interaction.

Preferably, the fibres 24 are arranged perpendicular to the bone grafting wall 16.

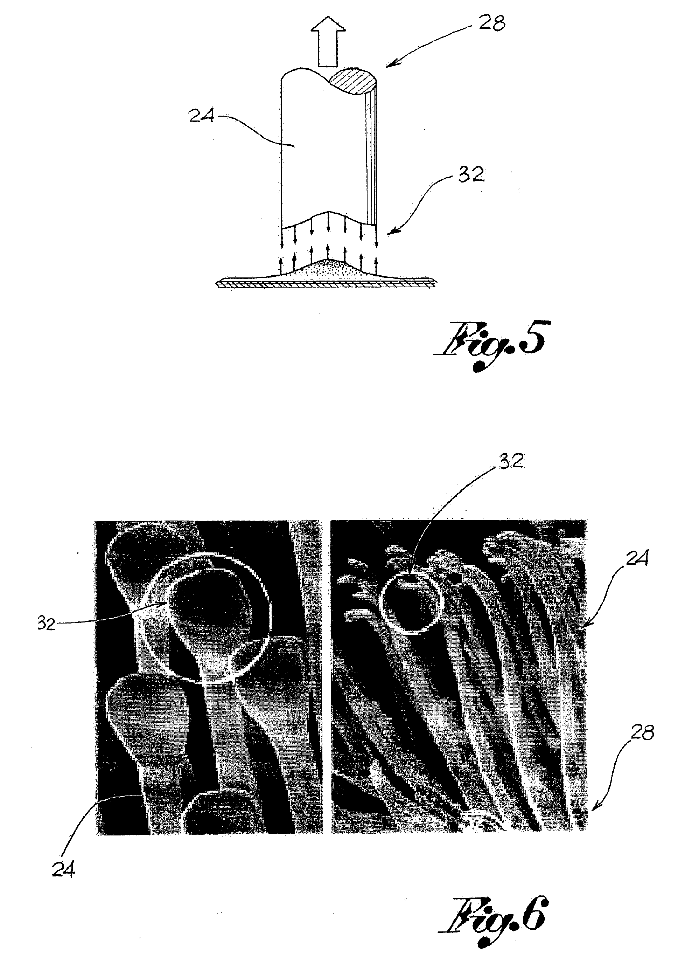

In other words, the fibres are cylindrical bodies having a radius of 90 to 110 nm and are positioned perpendicular to the bone grafting wall 16 so as to present a first prosthesis attachment base 28 in contact with said bone grafting wall 16 and a second, free, bone grafting base 32, opposite the first base 28, able to attract the bone cells to encourage grafting and growth on said wall 16 (figures 5-6).

Preferably, said fibres 24 are positioned, around said recesses 20 so as to define the edges.

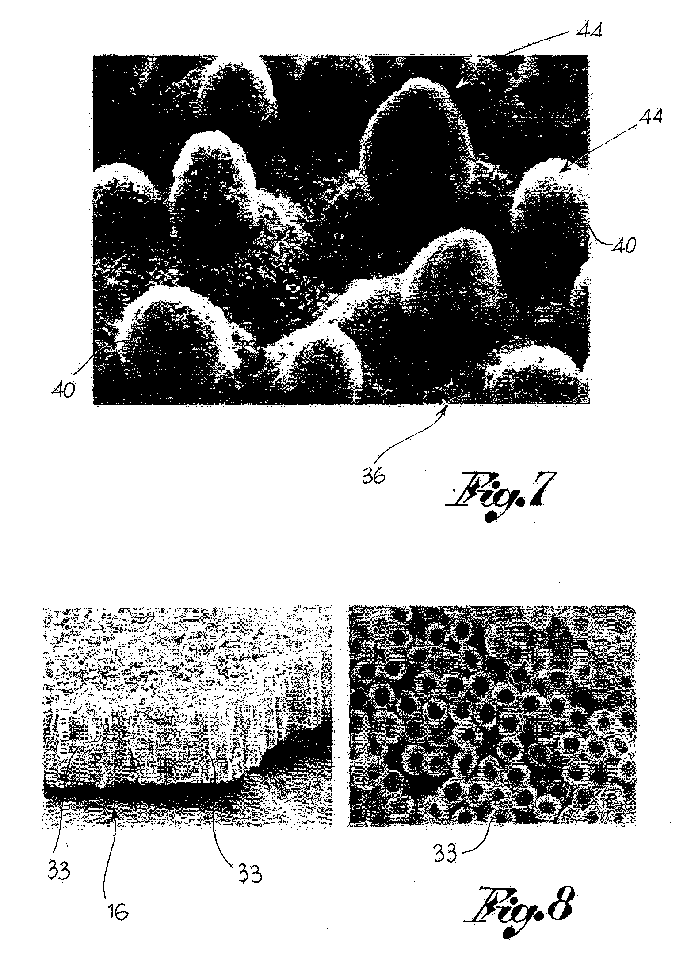

According to a further embodiment, the bone grafting wall 16 comprises nano-tubes 33, that is hollow cylindrical bodies, positioned preferably perpendicular to the bone grafting wall 16 (figure 8).

According to one embodiment said nano-tubes 33 have a diameter of 40 - 120 nm; preferably said diameter is 80 nm. Said nano-tubes 33 have a length of 200 - 600 nm; preferably said length is 400 nm.

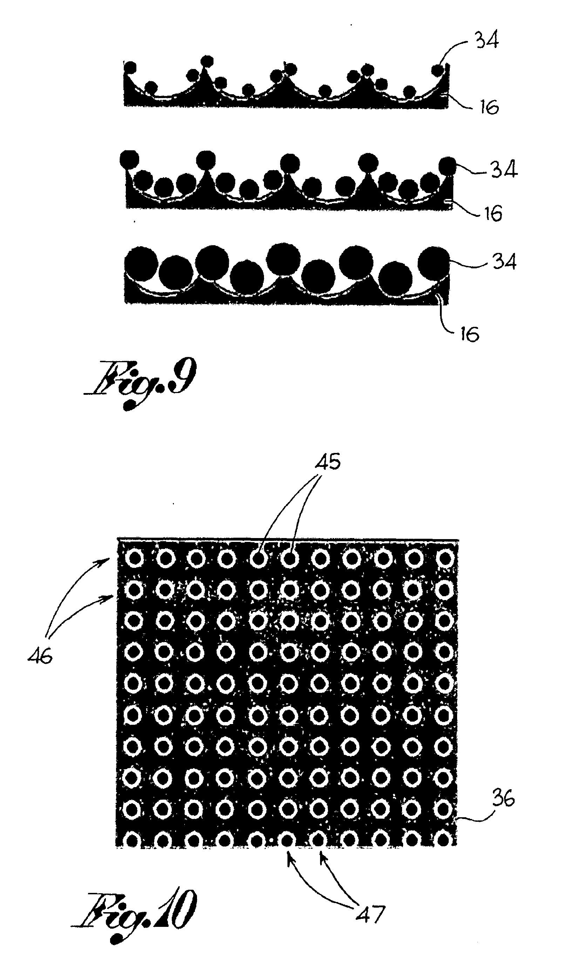

According to a further embodiment, the bone grafting wall 16 comprises nano-nodules 34, substantially spherical, positioned on said wall so as to improve bone grafting (figure 9). Preferably, said nano-nodules have a diameter of 50 - 500 nm. Even more preferably, said diameter of the nano-nodules is equal to 300 nm.

For example said structure comprising nano-nodules may be made using a PVD (physical vapour deposition) technique, such as sputtering.

According to a further embodiment, the bone grafting wall 16 comprises a sequence of amino acids, so as to encourage the implantation of the osteoblasts. For example, said sequence of amino acids comprises type RGD (Arg-Gly-Asp) amino acids. For example said sequence is contained in the proteins fibronectin, vitronectin, fibrinogen, nestin and collagen.

Advantageously, the primary attachment element 12 comprises at least one bone separation wall 36, said bone separation wall 36 being hydrophobic so as to oppose osteo-integration with the spongy tissue of the bone.

According to one possible embodiment, the bone separation wall 36 comprises a grid of nano-cones 40 suitable to oppose cellular adhesion, said nano-cones having tapered portions 44 directly facing the associable bone (figure 7).

The tapered morphology of the nano-cones 40 reduces the points of contact with the bone cells and thereby discourages the implantation of the prosthesis 4 in the bone.

According to one embodiment the nano-cones 40 comprise wax crystals which increase the hydrophobic nature of the bone separation wall 36.

According to the present invention, the bone separation wall 36 comprises a plurality of cavities 45 having a depth of 50 - 200 nm (figure 10) and a diameter of 60 - 240 nm. Preferably, the depth of the cavities 45 is 100 nm, while the diameter is 120 nm. Preferably, said cavities 45 are arranged in a regular grid shape; in other words said cavities 45 are aligned and arranged in rows 46 and columns 47.

According to a possible and non-limiting embodiment of the present invention, the prosthesis body 8 is a glenoid body suitable for interfacing with the head of a humerus (figure 2).

According to a possible embodiment of the present invention, the primary attachment element 12 comprises at least one pin 48 suitable for being inserted inside a bone so as to position itself in contact with the spongy tissue 14 inside the bone.

As may be appreciated from the description, the prosthesis according to the invention makes it possible to overcome the drawbacks presented in the prior art.

In particular, the prosthesis of the present invention is able to ensure rapid osteo-integration in correspondence of the contact surfaces with the bone suitable to ensure an efficient secondary stability of the prosthesis.

The stable and safe attachment of the prosthesis is thereby assured for the patient.

At the same time, the appendages suitable to create the primary stability of the prosthesis are made in such a way as to oppose osteo-integration.

In such manner, after separating the surfaces of the prosthesis body osteo-integrated with the bone cortex, the orthopaedic prosthesis may be explanted in a minimally invasive manner, without creating significant damage to the spongy bone tissue.

In fact, the spongy tissue forms a minimal or negligible graft on the attachment portions of the prosthesis and therefore, during explants, significant damage of said spongy tissue is not caused.

Thanks to the prosthesis of the present invention, prostheses can be serviced even repeatedly, while conserving the bone tissue.

It should be noted that the conformation of the prosthesis according to the present invention, is suitable to oppose, in localised portions, the osteo-integration of the prosthesis, at the same time preventing side effects from occurring, such as inflammation or rejection phenomena of the prosthesis.

A person skilled in the art may make numerous modifications and variations to the prosthesis described above so as to satisfy contingent and specific requirements.

As already described, the present invention is not limited to a specific type of orthopaedic prosthesis but may be referred to any orthopaedic prosthesis suitable to be implanted and subsequently explanted to perform servicing.

The prosthesis may be made in any biocompatible material so long as provided with at least one bone grafting wall and at least one bone separation wall as described above.

Merely by way of example, the present invention may be applied to vertebral body prostheses, hip prostheses, knee prostheses and the like.

These and other embodiment variations are all contained within the sphere of protection as defined by the appended claims.

## Claims

1. Orthopaedic prosthesis (4) comprising
- a prosthesis body (8) suitable for interfacing with at least one bony tissue,
- at least one primary attachment element (12) for the primary mechanical attachment of the orthopaedic prosthesis (4) inside said bony tissue,
wherein
- the prosthesis body (8) comprises at least one bone grafting wall (16) suitable, in an implant configuration, to be placed in contact with the bony tissue, said bone grafting wall (16) being suitable to improve the osteo-integration of the prosthesis (4) with the associable bony tissue,
- the primary attachment element (12) comprises at least one bone separation wall (36), said bone separation wall (36) being suitable to oppose osteo-integration with the bony tissue,
**characterised by** the fact that
the bone separation wall (36) comprises a plurality of cavities (45) having a depth of 50 - 200 nm and a diameter of 60 - 240 nm.

2. Prosthesis (4) according to claim 1, wherein said bone grafting wall (16) is hydrophilic and said bone separation wall (36) is hydrophobic.

3. Prosthesis (4) according to claim 1 or 2, wherein the bone grafting wall (16) comprises a surface grid having recesses (20) of a depth of 14 - 29 nm.

4. Prosthesis (4) according to claim 3, wherein said recesses (20) are blind holes not communicating with each other.

5. Prosthesis (4) according to any of the previous claims, wherein the bone grafting wall (16) comprises fibres (24) having a radius of 90 - 110 nm.

6. Prosthesis (4) according to any of the previous claims, wherein the bone grafting wall (16) comprises fibres (24) having a radius of 100 nm and wherein said fibres (24) are positioned substantially perpendicular to the bone grafting wall (16) so as to present a first prosthesis attachment base (28) in contact with the bone grafting wall (16) and a second, free, bone grafting base (32), opposite the first prosthesis attachment base (28), able to attract the bone cells encouraging grafting and growth on the bone grafting wall (16) itself.

7. Prosthesis (4) according to claim 5 or 6, wherein said fibres (24) are positioned around said recesses (20) so as to define the edges.

8. Prosthesis (4) according to any of the previous claims, wherein the bone grafting wall (16) comprises nano-tubes (33), in the shape of hollow cylindrical bodies, having a diameter of 40 - 120 nm and a length of 200 - 600 nm.

9. Prosthesis (4) according to any of the previous claims, wherein the bone grafting wall (16) comprises nano-nodules (34), substantially spherical and having a diameter of 50 - 500 nm.

10. Prosthesis (4) according to any of the previous claims, wherein the bone grafting wall (16) comprises a sequence of amino acids, wherein said amino acids are type RGD (Arg-Gly-Asp) amino acids.

11. Prosthesis (4) according to any of the previous claims, wherein the bone separation wall (36) comprises a grid of nano-cones (40) suitable to oppose cellular adhesion.

12. Prosthesis (4) according to claim 11, wherein said nano-cones (40) have tapered portions (44) directly facing the associable bone tissue.

13. Prosthesis (4) according to claim 11 or 12, wherein said nano-cones (40) comprise wax crystals.

14. Prosthesis (4) according to any one of previous claims, wherein said cavities (45) are aligned and arranged in rows (46) and columns (47) in a regular grid form.

15. Prosthesis (4) according to any of the previous claims, wherein the primary attachment element (12) comprises at least one pin (48) suitable for being inserted inside a bone so as to position itself in contact with a spongy tissue (14) inside the bone.

## Patentansprüche

1. Orthopädische Prothese (4), die:
- einen Prothesenkörper (8), geeignet für die Verbindung mit mindestens einem Knochengewebe,
- mindestens ein primäres Befestigungselement (12) für die primäre mechanische Befestigung der orthopädischen Prothese (4) innerhalb des genannten Knochengewebes, umfasst,
wobei
- der Prothesenkörper (8) mindestens eine Knochentransplantatwand (16) umfasst, die geeignet ist, in einer Implantatkonfiguration am Knochengewebe anzuliegen, wobei die genannte Knochentransplantatwand (16) geeignet ist, die Knochenintegration der Prothese (4) in das angrenzende Knochengewebe zu verbessern,
- das primäre Befestigungselement (12) mindestens eine Knochentrennwand (36) umfasst, wobei die genannte Knochentrennwand (36) geeignet ist, der Knochenintegration in das Knochengewebe entgegenzuwirken,
was **dadurch gekennzeichnet ist, dass**
die Knochentrennwand (36) eine Vielzahl an Hohlräumen (45) mit einer Tiefe von 50 - 200 nm und einem Durchmesser von 60 - 240 nm umfasst.

2. Prothese (4) nach Anspruch 1, wobei die genannte Knochentransplantatwand (16) hydrophil und die genannte Knochentrennwand (36) hydrophob ist.

3. Prothese (4) nach Anspruch 1 oder 2, wobei die Knochentransplantatwand (16) ein Oberflächengitter mit 14 - 29 nm tiefen Vertiefungen (20) umfasst.

4. Prothese (4) nach Anspruch 3, wobei die genannten Vertiefungen (20) Blindlöcher sind, die nicht miteinander in Verbindung stehen.

5. Prothese (4) nach einem der vorhergehenden Ansprüche, wobei die Knochentransplantatwand (16) Fasern (24) mit einem Radius von 90 - 110 nm umfasst.

6. Prothese (4) nach einem der vorhergehenden Ansprüche, wobei die Knochentransplantatwand (16) Fasern (24) mit einem Radius von 100 nm umfasst und wobei die genannten Fasern (24) im Wesentlichen senkrecht zu der Knochentransplantatwand (16) angeordnet sind, um eine erste Prothesenbefestigungsbasis (28), die an der Knochentransplantatwand (16) anliegt, zu bilden, sowie eine zweite, freiliegende Knochentransplantatsbasis (32) gegenüber der ersten Prothesenbefestigungsbasis (28), welche die Knochenzellen anlocken kann und dadurch den Knochenaufbau und das Wachstum an der Knochentransplantatwand (16) selbst fördert.

7. Prothese (4) nach Anspruch 5 oder 6, wobei die genannten Fasern (24) rund um die genannten Vertiefungen (20) angeordnet sind, um die Ränder festzulegen.

8. Prothese (4) nach einem der vorhergehenden Ansprüche, wobei die Knochentransplantatwand (16) Nanoröhrchen (33) in der Form von zylindrischen Hohlkörpern mit einem Durchmesser von 40 - 120 nm und einer Länge von 200 - 600 nm umfasst.

9. Prothese (4) nach einem der vorhergehenden Ansprüche, wobei die Knochentransplantatwand (16) Nanoknoten (34) umfasst, die im Wesentlichen kugelförmig sind und einen Durchmesser von 50 - 500 nm aufweisen.

10. Prothese (4) nach einem der vorhergehenden Ansprüche, wobei die Knochentransplantatwand (16) aus einer Reihe von Aminosäuren besteht, wobei die genannten Aminosäuren vom Typ RGD (Arg-Gly-Asp) sind.

11. Prothese (4) nach einem der vorhergehenden Ansprüche, wobei die Knochentrennwand (36) ein Gitter aus Nanokegeln (40) umfasst, die geeignet sind, der Zellanhaftung entgegenzuwirken.

12. Prothese (4) nach Anspruch 11, wobei die genannten Nanokegel (40) direkt gegenüber vom angrenzenden Knochengewebe sich verjüngende Teile (44) aufweisen.

13. Prothese (4) nach Anspruch 11 oder 12, wobei die genannten Nanokegel (40) Wachskristalle umfassen.

14. Prothese (4) nach einem der vorhergehenden Ansprüche, wobei die genannten Hohlräume (45) in Reihen (46) und Säulen (47) in regelmäßiger Gitterform angeordnet sind.

15. Prothese (4) nach einem der vorhergehenden Ansprüche, wobei das primäre Befestigungselement (12) mindestens einen Stift (48) umfasst, der sich in einen Knochen einführen lässt, sodass er mit der Spongiosa (14) innerhalb des Knochens in Kontakt steht.

## Revendications

1. Prothèse orthopédique (4) comprenant
- un corps de prothèse (8) adapté pour faire l'interface avec au moins un tissu osseux,
- au moins un élément de fixation primaire (12) pour la fixation mécanique primaire de la prothèse orthopédique (4) dans ledit tissu osseux,
dans laquelle
- le corps de prothèse (8) comprend au moins une paroi de greffe osseuse (16) adaptée, dans une configuration d'implant, pour être mise en contact avec le tissu osseux, ladite paroi de greffe osseuse (16) étant adaptée pour améliorer l'ostéo-intégration de la prothèse (4) avec le tissu osseux concerné,
- l'élément de fixation primaire (12) comprend au moins une paroi de séparation osseuse (36), ladite paroi de séparation osseuse (36) étant adaptée pour résister à l'ostéo-intégration avec le tissu osseux,
**caractérisée en ce que**
la paroi de séparation osseuse (36) comprend une pluralité de cavités (45) présentant une profondeur de 50 - 200 nm et un diamètre de 60 - 240 nm.

2. Prothèse (4) selon la revendication 1, dans laquelle ladite paroi de greffe osseuse (16) est hydrophile et ladite paroi de séparation osseuse (36) est hydrophobe.

3. Prothèse (4) selon la revendication 1 ou 2, dans laquelle la paroi de greffe osseuse (16) comprend un maillage de surface présentant des renfoncements (20) d'une profondeur de 14 - 29 nm.

4. Prothèse (4) selon la revendication 3, dans laquelle lesdits renfoncements (20) sont des trous borgnes ne communiquant pas les uns avec les autres.

5. Prothèse (4) selon l'une quelconque des revendications précédentes, dans laquelle la paroi de greffe osseuse (16) comprend des fibres (24) présentant un rayon de 90 - 110 nm.

6. Prothèse (4) selon l'une quelconque des revendications précédentes, dans laquelle la paroi de greffe osseuse (16) comprend des fibres (24) présentant un rayon de 100 nm et dans laquelle lesdites fibres (24) sont positionnées substantiellement perpendiculairement à la paroi de greffe osseuse (16), de manière à présenter une première base de fixation de prothèse (28) en contact avec la paroi de greffe osseuse (16) et une deuxième base de greffe osseuse (32) libre, opposée à la première base de fixation de prothèse (28), capable d'attirer les cellules osseuses favorisant la greffe et la croissance sur la paroi de greffe osseuse (16) elle-même.

7. Prothèse (4) selon la revendication 5 ou 6, dans laquelle lesdites fibres (24) sont positionnées autour desdits renfoncements (20) de manière à définir les bords.

8. Prothèse (4) selon l'une quelconque des revendications précédentes, dans laquelle la paroi de greffe osseuse (16) comprend des nano-tubes (33) sous la forme de corps cylindriques creux présentant un diamètre de 40 - 120 nm et une longueur de 200 - 600 nm.

9. Prothèse (4) selon l'une quelconque des revendications précédentes, dans laquelle la paroi de greffe osseuse (16) comprend des nano-nodules (34), substantiellement sphériques et présentant un diamètre de 50 - 500 nm.

10. Prothèse (4) selon l'une quelconque des revendications précédentes, dans laquelle la paroi de greffe osseuse (16) comprend une séquence d'acides aminés, lesdits acides aminés étant des acides aminés du type RGD (Arg-Gly-Asp).

11. Prothèse (4) selon l'une quelconque des revendications précédentes, dans laquelle la paroi de séparation osseuse (36) comprend un réseau de nano-cônes (40) adaptés pour résister à l'adhérence cellulaire.

12. Prothèse (4) selon l'une quelconque des revendications précédentes, dans laquelle lesdits nano-cônes (40) présentent des portions effilées (44) directement tournées vers le tissu osseux concerné.

13. Prothèse (4) selon l'une des revendications 11 ou 12, dans laquelle lesdits nano-cônes (40) comprennent des cristaux de cire.

14. Prothèse (4) selon l'une quelconque des revendications précédentes, dans laquelle lesdites cavités (45) sont alignées et disposées en rangées (46) et en colonne (47) sous la forme d'une grille ordinaire.

15. Prothèse (4) selon l'une quelconque des revendications précédentes, dans laquelle l'élément de fixation primaire (12) comprend au moins une broche (48) adaptée pour être insérée à l'intérieur d'un os, de manière à se positionner en contact avec un tissu spongieux (14) à l'intérieur de l'os.
